# EUROPEAN PATENT SPECIFICATION

(11) **EP 4 156 091 B1**
(45) Date of publication and mention of the grant of the patent: **18.03.2026**
(21) Application number: 21198958.7
(22) Date of filing: 24.09.2021
(51) Int. Cl.: G06T 7/00

(54) **COMPUTER-IMPLEMENTED METHOD FOR EVALUATING AN IMAGE DATA SET OF AN IMAGED REGION, EVALUATION DEVICE, IMAGING DEVICE, COMPUTER PROGRAM AND ELECTRONICALLY READABLE STORAGE MEDIUM**
COMPUTERIMPLEMENTIERTES VERFAHREN ZUR AUSWERTUNG EINES BILDDATENSATZES EINES ABGEBILDETEN BEREICHS, AUSWERTUNGSVORRICHTUNG, BILDGEBUNGSVORRICHTUNG, COMPUTERPROGRAMM UND ELEKTRONISCH LESBARES SPEICHERMEDIUM
PROCÉDÉ MIS EN UVRE PAR ORDINATEUR D'ÉVALUATION D'UN ENSEMBLE DE DONNÉES D'IMAGE D'UNE RÉGION IMAGÉE, DISPOSITIF D'ÉVALUATION, DISPOSITIF D'IMAGERIE, PROGRAMME INFORMATIQUE ET SUPPORT D'INFORMATIONS LISIBLE ÉLECTRONIQUEMENT

(43) Date of publication of application: 29.03.2023
(73) Proprietor: Siemens Healthineers AG, 91301 Forchheim (DE)
(72) Inventor: Allmendinger, Thomas, 91301 Forchheim (DE); Grimmer, Rainer, 91056 Erlangen (DE); Schwemmer, Chris, 91301 Forchheim (DE)
(74) Representative: Siemens Healthineers Patent Attorneys

(56) References cited:
- US-A1- 2020 375 564
- YANG GUANYU ET AL: "Automatic coronary calcium scoring using noncontrast and contrast CT images", MEDICAL PHYSICS, AIP, MELVILLE, NY, US, vol. 43, no. 5, 31 May 2016 (2016-05-31), pages 2174 - 2186, XP012206620, ISSN: 0094-2405, [retrieved on 19010101], DOI: 10.1118/1.4945045

## Description

The invention concerns a computer-implemented method for evaluating an image data set of an imaged region, in particular of a patient, wherein, from the imaged data set, different processed data sets having different image data content are determinable by image processing. The invention further concerns an evaluation device, an imaging device, a computer program and an electronically readable storage medium.

Advanced imaging techniques, in particular regarding computed tomography and magnetic resonance, provide an increasing amount of information regarding an imaged region, in particular regarding medical imaging, which often is a foundation of diagnostics. However, this information often cannot be directly seen from the acquired image data set, such that evaluation algorithms applied for evaluation of the image data set become more important, in particular in clinical routine. Some of these evaluation algorithms also employ artificial intelligence, in particular by comprising trained functions, for example neural networks. Evaluation algorithms, due to complexity, are usually developed to use one data set as input data, in particular the, for example pre-processed, image data set itself. For example, trained functions may be trained on a certain type of data set as input data.

Since, traditionally, evaluation algorithms, in particular for medical image data, work on single data sets, for example one reconstructed computed tomography volume with specific acquisition and reconstruction parameters, the information that the evaluation algorithm should extract should be visible in the single input data set. For example, an algorithm for detecting landmarks is required to run on data where these landmarks are visible, for example data acquired using a contrast agent. However, if an evaluation question requires different data, for example without contrast agent or functional image data that does not show any anatomy, such a landmark detection algorithm could not be used. Hence, evaluation is limited to the image information available in a certain data set. If several image data sets from different acquisitions were to be used, they would have to be registered, since those image data sets would not be aligned. On the other hand, from a clinical perspective, often both a contrasted and a non-contrasted scan are acquired, but it would be desirable to skip one scan to reduce the x-ray dose for the patient.

If only a single data set, for example image data set or processed data set, is used for an evaluation algorithm, the possibilities are reduced. For example, an automatic coronary calcium scoring requires non-contrasted input data to reliably identify classifications in the image data set. To assign these classifications to certain blood vessels, a respective segmentation would be highly beneficial, which is, however, strongly limited on non-contrasted data. Hence, the accuracy of such an assignment is also limited. Comparable problems arise in automatic heart valve calcium scoring, wherein, additionally, the heart valves are not aligned with axial images derivable from the image data set, while a valve-oriented view would be highly desirable for reading. Finding such a view is difficult on non-contrasted data, which is an additional problem to assigning classifications to valve leaflets.

Regarding the problem of spine and rib unfolding, non-contrasted data is required to reliably identify the spinal channel. On a contrasted acquisition, differentiation between the contrasted blood vessels and the spinal channel would be very difficult. In some cases, evaluations are not even possible using a single data set, for example for automatic anatomy-based image reformations for non-anatomical image data.

To solve this problem, it was proposed to acquire two image data sets and perform a registration. However, workflow and registration quality issues arise. For example, in automatic coronary and/or valve calcium scoring, the non-contrasted scan is usually performed before the contrasted scan, so that the latter is not available at the same time. In addition, due to the movement of the heart and the patient and the difficulty of finding anatomical landmarks in the non-contrasted data, registration is difficult and may lead to imperfect results, in particular artifacts. In another example, namely automatic anatomy-based image reformations for non-anatomical image data, due to the movement of the target anatomy and the patient and the difficulty of finding anatomical landmarks in non-anatomical image data, registration is difficult and may also lead to artifacts. Finally, regarding the automatic determination of the extra-cellular volume (ECV) or late enhancement in the myocardium, a segmentation of the blood pool and the myocardial segments based on a prior contrasted scan is required in addition to an extra non-contrast scan taken before for determination of the baseline.

The document US 20200375564 A1 discloses a method for myocardial computed tomography (CT) perfusion image synthesis based on spectral CT data.

It is an object of the invention to provide improved evaluation quality and improved reliability of quantitative evaluation results.

This object is achieved by providing a computer-implemented method according to claim 1, an evaluation device according to claim 12, an imaging device according to claim 13, a computer program according to claim 14, and an electronically readable storage medium according to claim 15. Advantageous embodiments are described in the dependent claims.

In a computer-implemented method for evaluating an image data set of an imaged region according to claim 1, according to the invention, an image data set from which different processed data sets having different image data content are determinable by image processing is used. To determine quantitative evaluation result data describing at least one dynamic and/or static feature of the imaged region by applying an evaluation algorithm, the method comprises
- determining at least two processed data sets having different image data content from the image data set,
- applying a first sub-algorithm of the evaluation algorithm to a first of the processed data sets to determine a first intermediate result relating to the image data content of the first processed data set,
- applying a second sub-algorithm of the evaluation algorithm to a second of the processed data sets to determine a second intermediate result relating to the image data content of the second processed data set, and
- determining the quantitative evaluation data by a third sub-algorithm of the evaluation algorithm, wherein the third sub-algorithm uses both the first and the second intermediate results as input data.

According to the invention, the image data set is an angiography data set and at least one of the processed datasets is a virtual non iodine image and in the non-iodine image, an identification and quantification of calcium is performed to yield the identification and quantification of calcium as second intermediate result.

Quantitative evaluation result data may, for example, comprise physical quantities not directly obvious/visible from the image data and/or non-localized physical quantities and/or physical quantities relating to semantical entities of the imaged region, for example certain anatomical features of a patient. In particular, the method is applied in medical imaging, such that the imaged region may be or comprise a region of interest of the patient. The image data set is preferably three-dimensional. The image data set is a computed tomography data set. While most examples discussed here will be related to medical imaging, it is noted that the principles described herein may also be applied in other applications, for example material testing.

From the image data set, different processed data sets having different image data content may be determinable. Different image data content means that different properties of the imaged region are visible in or derivable from the respective processed data sets. Advanced imaged processing techniques allow a plurality of processing variants to emphasize certain image contents/properties of the image region or, in particular, even allow their visualization. Besides using different filter kernels in computed tomography to create clearly different image impressions, a large plurality of processed data sets is conceivable using spectral computed tomography, which may also be called multi energy computed tomography or generally multi energy imaging.

The image data set is a multi energy computed tomography data set. In multi energy computed tomography, by applying corresponding image processing, image properties may be fundamentally changed, such that comparison/similarity of the resulting processed data sets may not even be provided. In particular, monoenergetic images may be determined as processed data sets, while, preferably, at least one of the processed data sets may be determined based on a material decomposition. In a material decomposition, different materials in the imaged region can be extracted or differently emphasized or suppressed.

In other words, using spectral computed tomography image data sets, it is possible to reconstruct very different image impressions from the same acquired image data set. For example, certain materials may be suppressed completely or their representation may be enhanced.

In particular, the imaged data set may be acquired using source-based and/or detector-based multi energy computed tomography, in particular using a counting x-ray detector. Source-based methods include, for example, dual layer computed tomography and kV switching technologies. However, since in source-based technologies, time differences may occur, even if they are small, detector-based computed tomography is preferred. Here, the processed data sets will be perfectly registered to each other since they have been acquired at the same time. In particularly preferred embodiments, a counting x-ray detector is used, which allows to separately measure single events and their x-ray photon energy, so that, for example, each single event may be sorted into one of multiple energy slots.

If the multi energy computed tomography image data set is an angiography data set, at least one of the processed data sets may be chosen from the group comprising a virtual non-contrast image, an iodine concentration image, a functional, in particular perfusion, image, a monoenergetic image, a virtual non-calcium image, and a virtual non-iodine image. For angiography, usually, an iodine contrast agent is used. However, techniques for image processing comprise determining a virtual non-contrast image from a iodine contrasted scan, which mimics a native non-contrast scan. Furthermore, iodine concentration images providing a quantitative iodine map of the individual voxels may also be determined. Functional images, for example regarding perfusion of tissue, may be derived from late enhancement contrasted image data sets. Additionally, monoenergetic images, in particular kV images at different kV level settings may be calculated. For example, very high kV images enable a view into metallic objects and low kV settings provide images with high anatomical detail. In a virtual non-calcium reconstruction, a non-calcium or calcium-subtracted image representation is provided. A virtual non-iodine image is similar to a virtual non-contrast image, however, the Hounsfield unit (HU) values of calcium or bone structures are preserved, such that such images may, for example, be used for calcium scoring.

While many examples discussed here may refer to coronary angiography, of course, other applications and medical imaging are also conceivable. For example, regarding liver tumors, contrasted computed tomography scans may also be performed to, for example, assess the blood perfusion of the tumor. This is particularly important regarding therapy monitoring, where, for example, as quantitative evaluation data, relative perfusion of the tumor and the liver may be determined.

Generally, in the invention, the use of at least two processed data sets having different image data content and the combination of respective evaluation results leads to an improved quality of the final results, in particular by combining anatomical information with other, for example functional, information. In particular when using multi energy computed tomography, no registration between different representations of the imaged region is required, such that faster processing, less artifacts and in particular perfect spatial match is achieved. In particular, a separate scan in computer tomography can be avoided by generating virtual non-contrast data sets out of a contrasted acquisition. Generally, an enhanced amount of information is provided to the evaluation algorithm. Using multiple representations of the imaged region, that is multiple processed data sets, allows the determination of additional information which cannot be derived from only a single data set.

It is noted that, while two processed data sets are explicitly discussed as an example, it is also possible to use more than two processed data sets providing further intermediate results in addition to the first and second intermediate results, which may also be used by the subsequent third sub-algorithm as input data. In other embodiments, cascaded evaluations may also be possible, for example by combining at least one pair of intermediate results into another intermediate results, which may then be used as input data for a subsequent sub-algorithm. In other words, it can be generally said that one or more first sub-algorithms, second sub-algorithms and third sub-algorithms may be applied to one or more processed data sets, second processed data sets, or one or more first and second intermediate results, respectively. In preferred embodiments, the first intermediate result is or describes a segmentation result regarding multiple segmented features, wherein the third sub-algorithm assigns data of the second intermediate result to segmented features to yield quantitative segmented feature-specific evaluation results. Regarding medical imaging, for example, the first intermediate result may describe anatomy of interest, for example blood vessels in a blood vessel tree of a patient. The second intermediate result may, for example, relate to a quantitative concentration of material, for example calcium, such that it can be determined how much of the material is present in different segmented features, such that quantitative evaluation result data may be provided as amounts or concentrations of material in certain segmented features. Of course, also other quantitative second intermediate results may be assigned to segmented entities, for example perfusion values to certain tissue segments.

As already indicated, the segmented features may preferably be anatomical features and comprise vessels or vessel segments of a vessel tree and/or organs or organ segments. In a concrete embodiment, in multi energy computed tomography angiography, in a low kV monoenergetic image, the heart and its valves as well as the coronary arteries may be segmented and labelled. According to the invention, the second processed data set is a non-contrasted virtual non-iodine data set, in which an identification and quantification of calcium is performed to yield a respective second intermediate result. In combination, by using the third sub-algorithm correctly quantified calcium values correctly assigned to coronary vessels and/or heart valves can be determined. In another example, in a contrasted low kV representation as first processed data set, a myocardium segmentation may be performed, while the second processed data set may comprise a functional representation, from which perfused blood volume information can be deduced using the second sub-algorithm. In combination, the third sub-algorithm may output perfused blood volume information for different segments or sections of the myocardium, for example standardized 18 sections, as quantitative evaluation result data. In concrete embodiments, for example, the segmented myocardium may be used as a mask by the third sub-algorithm of the evaluation algorithm. It is noted that perfused blood volume information may, alternatively, also be extracted from a iodine data set as second processed data set.

In another example, a tumor and an organ, for example the liver, may be segmented in at least one first processed data set, wherein the second intermediate result may, again, be a perfusion value, for example blood volume perfusion information. The result of the third sub-algorithm and of the evaluation algorithm as a whole may then be quantitative perfusion values of the tumor relative the organ.

Generally speaking, in such embodiments, the first sub-algorithm may be a segmentation algorithm as in principle known in the art. The second sub-algorithm may determine quantitative per-voxel results or a segmentation result regarding a material or generally, physical quantity, wherein the third sub-algorithm may combine the segmentations of the first and second intermediate result.

In an especially preferred embodiment, the first intermediate result comprises a segmented vessel tree, wherein the third sub-algorithm also performs at least one fluid flow simulation in the segmented vessel tree to determine at least one fluid flow parameter as an evaluation result, wherein the simulation is at least partly parameterized using the second intermediate result. In particular, the segmented vessel tree may be a blood vessel tree, the fluid may be blood and the at least one fluid flow parameter may comprise a fractional flow reserve (FFR). In the state of the art, it has already been proposed to use segmented vessel trees for simulations, however, the quality of the simulation results may be reduced due to missing information. According to the invention, this missing information may be provided by the at least one second intermediate result, such that the first intermediate result provides the geometry of the vessel tree, in particular also a relevant organ like a heart, for example as a mesh, while further, in particular functional, information, that is simulation parameters, is provided in the second intermediate result. For example, in a iodine data set in angiography, the lumen of the vessels may be determined, while in a low kV monoenergetic image as second processed data set, a myocardium mass and other parameters may be determined. It should be noted that, in such an embodiment, a staged use of the principles discussed here may also be employed, such that, for example, a calcium image may also be determined as a processed data set, assisting in separating calcium from iodine and thus being able to better describe strongly calcified stenosis areas, which are difficult to segment, and/or include aneurysms into the simulation. In particular, other fluid flow parameters may also refer to such areas or aneurysms. In particularly preferred examples, additionally or alternatively, implants, for example stents, may be localized and/or precisely described based on high kV images, even allowing determination of fluid flow parameters, for example FFR, inside an implant, for example stent or stent graft.

In other preferred embodiments, at least a part of the first and at least a part of the second intermediate result may be used as quantitative input data to at least one disease value estimation of the third sub-algorithm. For example, the quantitative evaluation result data may comprise a disease score, in particular spatially resolved. Examples comprise calcium scores and a plaque classification. However, the disease value may also more concretely describe the disease, for example it may be determined whether a plaque is calcified, necrotic and/or to which share these classes apply. Such information may, for example, be deduced from monoenergetic data sets having as low energy as possible. In other embodiments, the disease value may also be a prediction value, for example quantify a percentage of a certain event occurring. In an example, from FFR values, risks for certain events and/or conditions may be quantitively calculated.

In advantageous embodiments, the third sub-algorithm additionally determines at least one two-dimensional output image visualizing the evaluation result data. Such output images may, in particular, also be derived from the image data set and/or at least one of the processed data sets. If a simulation is performed, of course, they may be completely newly generated. Such output images may provide a better understanding of the evaluation result data. In concrete embodiments, the orientation and/or view point and/or shown imaged region portion of the at least one output image based on the second processed data set and/or the second intermediate result is chosen based on the first intermediate result.

In concrete embodiments, for example, regarding calcification values, in particular calcification scores, the heart and valve orientation may be determined from the segmentation of the first processed data set and may define heart- and/or value-oriented views for displaying quantified calcium values in an output image. A heart-oriented view may, for example, comprise images are perpendicular to the short axis or the long axis of the heart. In particular, multiple output images along such axis may be provided. Of course, such heart-oriented views or valve-oriented views may also be used in other applications. For example, perfused blood volume information may be shown overlaid over the myocardium.

It is noted that another area of application of the principles described here is the assessment of implants. For example, if a stent or stent graft is implanted into a vessel, calcium may accumulate inside the stent. On the other hand, implants as strongly attenuating objects may be problematic in x-ray imaging. However, high kV representations, that is monoenergetic data sets, may clearly show the implant. This can be used to, for example, section-wise determine calcium or other occlusions inside the stent graft or stent, wherein, for example, the stent graft or stent may be removed from a processed data set while still using its position to evaluate materials inside the stent or stent graft. In particular, regarding output images, views into an implant, in particular a stent or stent graft, may be provided. If anatomical landmarks are determined from a low kV representation as first processed data set, such views into an implant may be oriented such that cross-sections of the vessel result as output images.

In embodiments of the invention, at least one sub-algorithm may comprise a trained function. While, of course, also sub-algorithms using no artificial intelligence may be employed, the principles of the current invention are particularly advantageous if at least one of the sub-algorithms is a trained function. In general, a trained function mimics cognitive functions that humans associate with other human minds. In particular, by training based on training data the trained function is able to adapt to new circumstances and to detect and extrapolate patterns.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted intuitively by several steps of training.

In particular, a trained function can comprise a neural network, a support vector machine, a decision tree and/or a Bayesian network, and/or the trained function can be based on k-means Clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

The invention further concerns an evaluation device for evaluating an image data set of an imaged region according to claim 12, wherein, from the image data set, different processed data sets having different image data content are determinable by image processing, the evaluation device comprising:
- a first interface for receiving the image data set,
- an image processing unit for determining at least two processed data sets having different image data content from the image data set,
- an evaluation unit for determining quantitative evaluation result data describing at least one dynamic and/or static feature of the imaged region by applying an evaluation algorithm, and
- a second interface for providing the evaluation result data,
wherein the evaluation unit comprises:
- a first subunit for applying a first sub-algorithm of the evaluation algorithm to a first of the processed data sets to determine a first intermediate result relating to the image data content of the first processed image data set,
- a second subunit for applying a second sub-algorithm of the evaluation algorithm to a second of the processed data sets to determine a second intermediate result relating to the image data content of the second processed data set, and
- a third subunit for determining the quantitative evaluation data by a third sub-algorithm of the evaluation algorithm, wherein the third sub-algorithm uses both the first and the second intermediate results as input data.

In particular, the evaluation device is configured to perform a method according to the invention. All features and remarks regarding the method according to the invention analogously apply to the evaluation device according to the invention. Preferably, the evaluation device may comprise at least one processor and at least one storage means and/or the functional units may be implemented at least partly by software and/or at least partly by hardware components.

An imaging device according to the invention has a control device comprising an evaluation device according to the invention. In particular, the imaging device may be a medical imaging device, for example a magnetic resonance device or, preferably, a computed tomography device. In particular regarding computed tomography, many acquisition techniques are known which result in image data sets, from which different processed data sets showing different image content can be determined. Regarding magnetic resonance devices, image data sets may be used which, for example in partial data sets, show different contrasts and/or from which processed data sets relating to different contrasts may be calculated. An exemplary embodiment from magnetic resonance imaging is the so-called Dixon technique, where partial data sets are acquired at different echo times to differentiate between different spin species, between which a chemical shift exists. For example, spins of protons bound in fat may be distinguished from spins of protons bound in water.

Providing the evaluation device as a part of an imaging device has the advantage of providing important information, in this case the high-quality quantitative evaluation result data, immediately where the image data set was acquired. However, it should be noted that the evaluation device may also be or be part of a viewing and/or evaluation work station, for example a PACS workstation (PACS - picture archiving and communication system).

A computer program according to the invention can be directly loaded into a storage means of an evaluation device and, if executed on the evaluation device, performs the steps of a method according to the invention. The computer program may be stored on an electronically readable storage medium according to the invention, which hence comprises control information comprising a computer program according to the invention, such that, when the electronically readable storage medium is used in an evaluation device, the evaluation device is configured to perform the steps of a method according to the invention. The electronically readable storage medium may be a non-transitory medium, for example a CD-ROM.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: a flowchart of a general embodiment of a method according to the invention,
- Fig. 2: the functional structure of an evaluation device according to the invention, and
- Fig. 3: a schematic view of an imaging device according to the invention.

In the following, embodiments of the invention are described with respect to a multi-energy computed tomography angiography data set as image data set to be evaluated. However, also other image data sets, from which processed data sets having different image content can be derived, can be used.

The image data set of these embodiments has been acquired in detector-based multi energy computed tomography by using a counting x-ray detector. That is, all image data have been acquired in one single acquisition, such that derived processed data sets are all registered to each other.

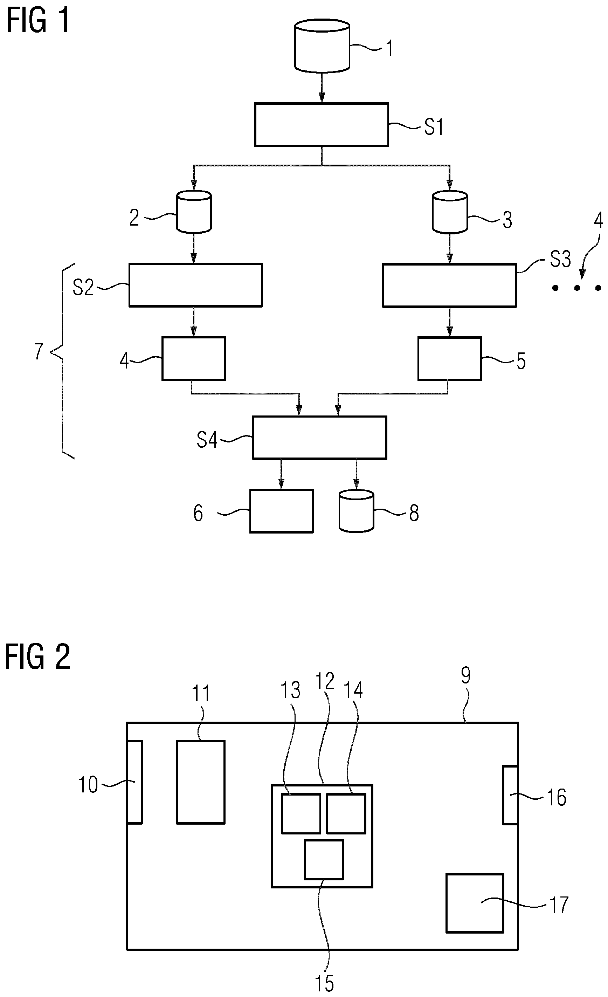

Fig. 1 shows a general flowchart of a method according to the invention. After the image data set one has been provided, in particular received via a first interface, in step S1, multiple processed data sets all having different image data content are derived from the image data set by image processing. For example, by using material decomposition, as principally known in the state of the art, virtual non-contrast images, iodine concentration images, functional images, virtual non-calcium images, virtual non-iodine images and the like can be determined. Additionally, or alternatively, monoenergetic images relating to certain energy intervals, for example defined energy slots or bins of the counting x-ray detector, may be determined. Each processed data set 2, 3 determined in this manner forms input data to at least one first or at least one second sub-algorithm of an evaluation algorithm configured to determine quantitative evaluation result data, for example calcium scores, FFR, perfused blood volume information and the like. In this embodiment, a first processed data set 2 is determined as input to a first sub-algorithm and a second processed data set 3 is determined as input data for a second sub-algorithm. However, this example is not limiting, and, as indicated by the dots 4, a larger number of first and/or second processed data sets 2, 3 and /or first and/or second sub-algorithms may be employed, in particular also in a stacked manner.

In a step S2, the first sub-algorithm is applied to the first processed data set 2, while in a step S3, the second sub-algorithm of the evaluation algorithm is applied to the second processed data set 3. In this manner, first and second intermediate results 4, 5 are determined. These intermediate results 4, 5 now both serve as input data for a third sub-algorithm of the evaluation algorithm, which is executed in step S4 to yield the quantitative evaluation result data **6.** Additionally, the output data of the third sub-algorithm and hence the evaluation algorithm formed by the first sub-algorithm, the second sub-algorithm and the third sub-algorithm, and indicated as reference number 7 may also comprise at least one two-dimensional output image 8, in particular series of output images 8, visualizing or explaining the quantitative evaluation result data 6.

In this manner, from the same image data set 1, multiple processed data sets 2, 3, each showing different image data content, are derived, intermediate results 4, 5 are determined therefrom and together used as input data for a third sub-algorithm in step S4 to provide additional information and improved quality of the evaluation result data 6 and the output images 9. Processed data sets 2, 3 are already registered, such that no additional registration process is required. The different processed data sets can be understood as different representations of the image data set, each including different pieces of information which, put together, allow to provide an improved and reliable evaluation result.

In a first concrete embodiment, the first processed data set 2 may be a contrasted low-energy representation, from which, by the first sub-algorithm, as the first intermediate result 4, segmentation and labelling information regarding a vessel tree and/or organs in the imaged region of a patient is determined. In coronary angiography, the first intermediate result 4 thus describes the position and orientation of the heart and its valves as well as the lumen, course and labelling of the coronary arteries. As a second processed data set 3, a non-contrasted virtual non-iodine image is determined. Here, the second sub-algorithm provides, as second intermediate result 5, identification and quantification of calcium in the imaged region. The third sub-algorithm uses both intermediate results 4, 5 as input and determines correctly quantified calcium values correctly assigned to coronary vessels a valve- and/or heart-oriented view, which view orientation and the corresponding positions may be determined from the first intermediate result. For example, a stack of two-dimensional MPR images may be determined along the long axis or the short axis of the heart.

In another embodiment or additionally, the or an additional second processed data set 3 may be a functional image, for example a late enhancement functional representation showing iodine, that is contrast agent, concentration in tissue, in particular the myocardium. From this functional representation, the second sub-algorithm may determine a perfused blood volume information as a second intermediate result 5, such that, after combination by the third sub-algorithm, for example perfused blood volume information may be shown overlaid over the myocardium in output images 8 in a heart-oriented view, while blood volume perfusion values may be quantitatively determined for different sections of the myocardium.

Furthermore, using a low energy monoenergetic image as the first processed data set 2 and a high-energy monoenergetic image as the second processed data set 3, anatomical landmarks and calcium information may be derived as first intermediate result 4 and the position and orientation of a stent or stent graft as intermediate result 5. Put together by the third sub-algorithm, a quantitative and visualized description of stent or stent graft occlusion by calcium can be derived in the manner of looking into the stent or stent graft.

In an especially advantageous concrete embodiment, the first processed data set 2 may be an iodine image, from which a segmented vessel tree is determined as first intermediate result 4. An additional processed data set 2 is a low-energy monoenergetic image, from which further anatomical features may be segmented such that a set of parameters relevant for the simulation of fluid flow in the vessel tree, for example the myocardium mass, may be determined. The third sub-algorithm then also performs a simulation of a blood flow in the vessel tree, which is parameterized using the second intermediate result 5. In this manner, at least one fluid flow parameter may be determined as evaluation result data, preferably at least the FFR (fractional flow reserve). In such embodiments, more than two processed data sets 2, 3 may be employed, for example by additionally using a virtual non-iodine image to detect calcifications and also take these into account in the simulation.

As a final concrete example, the first and second intermediate results 4, 5 may also be combined to determine at least one disease value using a respective disease value estimation in the third sub-algorithm. The disease value information may, in particular, comprise a trained function, such that, for example, a machine learning-based disease burden estimation is possible. In other examples, plaque quantification and classification may be performed.

But also generally, any of the first, second and third sub-algorithms may comprise trained functions, as already discussed above.

Fig. 2 shows the functional structure of an evaluation device 9 according to the invention. The evaluation device 9 comprises a first interface 10 for receiving the image data set 1. In an image processing unit 11, the processed data sets 2, 3 may be determined according to step S1. An evaluation unit 12 is provided for applying the evaluation algorithm 7 and comprises three subunits 13, 14 and 15, wherein the first subunit 13 executes the first sub-algorithm to determine the intermediate result 4, as described with regard to step S2, and the second subunit 14 executes the second sub-algorithm to determine the second intermediate result 5, as described with regard to step S3. Of course, as discussed above, multiple first and/or second subunits 13, 14 may also be provided and/or stacked structure of subunits 13, 14 and 15 may be present if the principles described herein are multiply used.

In the third subunit 15, the third sub-algorithm is performed to yield the quantitative evaluation result data 6 and optionally the at least one output image 8 described with respect to step S4. The evaluation result data 6 and the output images 8 may be provided via a second interface 16 of the evaluation device 9.

The evaluation device 9 may further comprise a storage means 17 for storing data temporally or permanently for later retrieval, for example image data sets 1, processed data sets 2, 3, intermediate results 4, 5, evaluation result data 6 and output images 9.

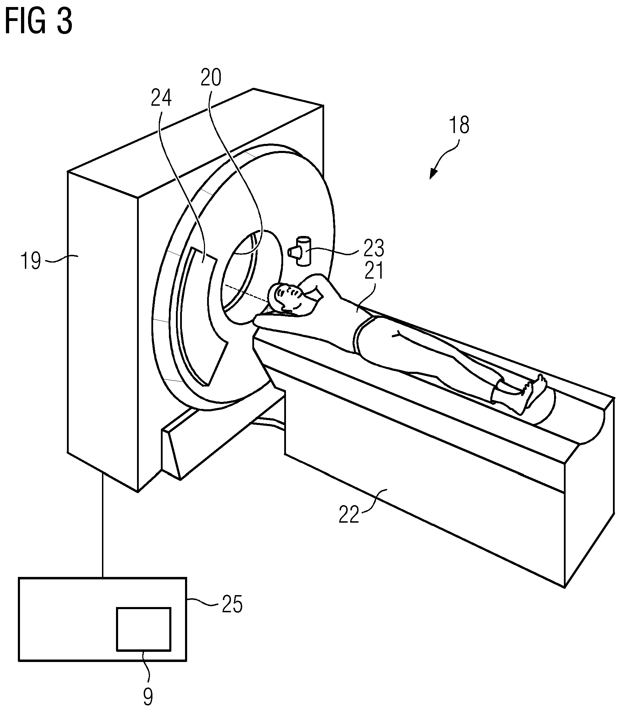

Fig. 3 schematically shows an imaging device 19 according to the current invention, in this case a computed tomography device. The imaging device 19 comprises a gantry 19 having a patient opening 20, into which a patient 21 may be introduced using a patient table 22. An acquisition assembly comprises an x-ray source 23 and an x-ray detector 24, in this case a counting x-ray detector 24, and may be rotated around the opening 20 and hence the patient to acquire projection images using different projection angles, from which an image data set 1 can be reconstructed.

The operation of the imaging device 18 is controlled by a control device 25, which, in this case, also comprises an evaluation device 9 according to the invention.

Although the present invention has been described in detail with reference to the preferred embodiment, the present invention is not limited by the disclosed examples from which the skilled person is able to derive other variations without departing from the scope of the invention.

## Claims

1. Computer-implemented method for evaluating a multi energy computed tomography image data set (1) of an imaged region, wherein, from the image data set (1), different processed data sets (2, 3) having different image data content are determinable by image processing, and quantitative evaluation result data (6) describing at least one dynamic and/or static feature of the imaged region is determined by applying an evaluation algorithm (7), the method comprising:
- determining at least two processed data sets (2, 3) having different image data content from the image data set (1),
- applying a first sub-algorithm of the evaluation algorithm (7) to a first of the processed data sets (2, 3) to determine a first intermediate result (4) relating to the image data content of the first processed data set (2),
- applying a second sub-algorithm of the evaluation algorithm (7) to a second of the processed data sets (2, 3) to determine a second intermediate result (5) relating to the image data content of the second processed data set (3),
- determining the quantitative evaluation result data (6) by a third sub-algorithm of the evaluation algorithm (7), wherein the third sub-algorithm uses both the first and the second intermediate results (4, 5) as input data,
wherein the image data set (1) is an angiography data set and the method is **characterized in that** at least one of the processed datasets (2, 3) is a virtual non iodine image and in the non-iodine image, an identification and quantification of calcium is performed to yield the identification and quantification of calcium as second intermediate result.

2. Method according to claim 1, wherein at least one of the processed data sets (2, 3) is determined based on a material decomposition and/or as a monoenergetic image.

3. Method according to claim 2, wherein the image data set (1) is acquired using source-based and/or detector based multi energy computed tomography, in particular using a counting x-ray detector (24).

4. Method according to claim 1 or 2, wherein the image data set (1) is an angiography data set and at least one of the processed datasets (2, 3) is chosen from the group comprising a virtual non contrast image, a iodine concentration image, a functional, in particular perfusion, image, a monoenergetic image, and a virtual non calcium image.

5. Method according to one of the preceding claims, wherein the first intermediate result (4) describes a segmentation result regarding multiple segmented features, wherein the third sub-algorithm assigns data of the second intermediate result (5) to segmented features to yield quantitative segmented feature-specific evaluation results.

6. Method according to claim 5, wherein the segmented features are anatomical features and comprise vessels or vessel segments of a vessel tree and/or organs or organ segments.

7. Method according to one of the preceding claims, wherein the first intermediate result (4) comprises a segmented vessel tree, wherein the third sub-algorithm performs at least one fluid flow simulation in the segmented vessel tree to determine at least one fluid flow parameter as an evaluation result, wherein the simulation is at least partly parametrized using the second intermediate result (5).

8. Method according to claim 7, wherein the segmented vessel tree is a blood vessel tree, the fluid is blood and the at least one fluid flow parameter comprises a fractional flow reserve.

9. Method according to one of the preceding claims, wherein at least a part of the first and at least a part of the second intermediate results (4, 5) are used as quantitative input data to at least one disease value estimation of the third sub-algorithm.

10. Method according to one of the preceding claims, wherein the third sub-algorithm additionally determines at least one two-dimensional output image (8) visualizing the evaluation result data (6).

11. Method according to claim 10, wherein the orientation and/or viewpoint and/or shown imaged region portion of the at least one output image (8) based on the second processed data set (3) and/or the second intermediate result (5) is chosen based on the first intermediate result (4).

12. Evaluation device (9) for evaluating a multi energy computed tomography image data set (1) of an imaged region, wherein, from the image data set (1), different processed data sets (2, 3) having different image data content are determinable by image processing, the evaluation device (9) comprising:
- a first interface (10) for receiving the image data set (1),
- an image processing unit (11) for determining at least two processed data sets (2, 3) having different image data content from the image data set (1),
- an evaluation unit (12) for determining quantitative evaluation result data (6) describing at least one dynamic and/or static feature of the imaged region by applying an evaluation algorithm (7),
- a second interface (16) for providing the evaluation result data (6),
wherein the evaluation unit (12) comprises:
- a first subunit (13) for applying a first sub-algorithm of the evaluation algorithm (7) to a first of the processed data sets (2, 3) to determine a first intermediate result (4) relating to the image data content of the first processed data set (2),
- a second subunit (14) for applying a second sub-algorithm of the evaluation algorithm (7) to a second of the processed data sets (2, 3) to determine a second intermediate result (5) relating to the image data content of the second processed data set (4),
- a third subunit (15) for determining the quantitative evaluation result data (6) by a third sub-algorithm of the evaluation algorithm (7), wherein the third sub-algorithm uses both the first and the second intermediate results (4, 5) as input data,
wherein the image data set (1) is an angiography data set and the device is **characterized in that** at least one of the processed datasets (2, 3) is a virtual non iodine image and in the non-iodine image, an identification and quantification of calcium is performed to yield the identification and quantification of calcium as second intermediate result.

13. Imaging device (18) with a control device (25) comprising an evaluation device (9) according to claim 12.

14. Computer program, which performs the steps of a method according to any of the claims 1 to 11 when the computer program is executed on an evaluation device (9).

15. Electronically readable storage medium, on which a computer program according to claim 14 is stored.

## Patentansprüche

1. Computerimplementiertes Verfahren zum Auswerten eines Mehrfachenergie-Computertomographie-Bilddatensatzes (1) einer abgebildeten Region, wobei aus dem Bilddatensatz (1) unterschiedliche verarbeitete Datensätze (2, 3) mit unterschiedlichem Bilddateninhalt durch Bildverarbeitung bestimmbar sind und quantitative Auswertungsergebnisdaten (6), die mindestens ein dynamisches und/oder statisches Merkmal der abgebildeten Region beschreiben, durch Anwenden eines Auswertungsalgorithmus (7) bestimmt werden, wobei das Verfahren Folgendes umfasst:
- Bestimmen von mindestens zwei verarbeiteten Datensätzen (2, 3), die einen anderen Bilddateninhalt als der Bilddatensatz (1) aufweisen,
- Anwenden eines ersten Unteralgorithmus des Auswertungsalgorithmus (7) auf einen ersten der verarbeiteten Datensätze (2, 3), um ein erstes Zwischenergebnis (4) zu bestimmen, das mit dem Bilddateninhalt des ersten verarbeiteten Datensatzes (2) in Beziehung steht,
- Anwenden eines zweiten Unteralgorithmus des Auswertungsalgorithmus (7) auf einen zweiten der verarbeiteten Datensätze (2, 3), um ein zweites Zwischenergebnis (5) zu bestimmen, das mit dem Bilddateninhalt des zweiten verarbeiteten Datensatzes (3) in Beziehung steht,
- Bestimmen der quantitativen Auswertungsergebnisdaten (6) durch einen dritten Unteralgorithmus des Auswertungsalgorithmus (7), wobei der dritte Unteralgorithmus sowohl das erste als auch das zweite Zwischenergebnis (4, 5) als Eingangsdaten verwendet, wobei der Bilddatensatz (1) ein Angiographiedatensatz ist, und das Verfahren **dadurch gekennzeichnet ist, dass**
mindestens einer der verarbeiteten Datensätze (2, 3) ein virtuelles Nicht-Iod-Bild ist, und dass in dem Nicht-Iod-Bild eine Identifikation und Quantifizierung von Calcium durchgeführt wird, um die Identifikation und Quantifizierung von Calcium als zweites Zwischenergebnis zu ergeben.

2. Verfahren nach Anspruch 1, wobei mindestens einer der verarbeiteten Datensätze (2, 3) basierend auf einer Materialzersetzung und/oder als ein monoenergetisches Bild bestimmt wird.

3. Verfahren nach Anspruch 2, wobei der Bilddatensatz (1) unter Verwendung quellenbasierter und/oder detektorbasierter Mehrfachenergie-Computertomographie, insbesondere unter Verwendung eines zählenden Röntgendetektors (24), erfasst wird.

4. Verfahren nach Anspruch 1 oder 2, wobei der Bilddatensatz (1) ein Angiographiedatensatz ist, und mindestens einer der verarbeiteten Datensätze (2, 3) ausgewählt ist aus der Gruppe, die ein virtuelles Nicht-Kontrastbild, ein Iod-Konzentrationsbild, ein funktionelles Bild, insbesondere Perfusionsbild, ein monoenergetisches Bild, und ein virtuelles Nicht-Calcium-Bild umfasst.

5. Verfahren nach einem der vorhergehenden Ansprüche, wobei das erste Zwischenergebnis (4) ein Segmentierungsergebnis hinsichtlich mehrerer segmentierter Merkmale beschreibt, wobei der dritte Unteralgorithmus Daten des zweiten Zwischenergebnisses (5) segmentierten Merkmalen zuweist, um quantitative segmentierte merkmalsspezifische Auswertungsergebnisse zu ergeben.

6. Verfahren nach Anspruch 5, wobei die segmentierten Merkmale anatomische Merkmale sind und Gefäße oder Gefäßsegmente eines Gefäßbaums und/oder Organe oder Organsegmente umfassen.

7. Verfahren nach einem der vorhergehenden Ansprüche, wobei das erste Zwischenergebnis (4) einen segmentierten Gefäßbaum umfasst, wobei der dritte Unteralgorithmus mindestens eine Fluidflusssimulation in dem segmentierten Gefäßbaum durchführt, um mindestens einen Fluidflussparameter als ein Auswertungsergebnis zu bestimmen, wobei die Simulation mindestens teilweise unter Verwendung des zweiten Zwischenergebnisses (5) parametrisiert wird.

8. Verfahren nach Anspruch 7, wobei der segmentierte Gefäßbaum ein Blutgefäßbaum ist, das Fluid Blut ist, und der mindestens eine Fluidflussparameter eine fraktionelle Flussreserve umfasst.

9. Verfahren nach einem der vorhergehenden Ansprüche, wobei mindestens ein Teil des ersten und mindestens ein Teil des zweiten Zwischenergebnisses (4, 5) als quantitative Eingangsdaten zu mindestens einer Krankheitswertschätzung des dritten Unteralgorithmus verwendet werden.

10. Verfahren nach einem der vorhergehenden Ansprüche, wobei der dritte Unteralgorithmus zusätzlich mindestens ein zweidimensionales Ausgabebild (8) bestimmt, das die Auswertungsergebnisdaten (6) visualisiert.

11. Verfahren nach Anspruch 10, wobei die Orientierung und/oder der Blickpunkt und/oder der gezeigte abgebildete Regionsabschnitt des mindestens einen Ausgabebildes (8) basierend auf dem zweiten verarbeiteten Datensatz (3) und/oder das zweite Zwischenergebnis (5) basierend auf dem ersten Zwischenergebnis (4) ausgewählt wird/werden.

12. Auswertungsvorrichtung (9) zum Auswerten eines Mehrfachenergie-Computertomographie-Bilddatensatzes (1) einer abgebildeten Region, wobei aus dem Bilddatensatz (1) unterschiedliche verarbeitete Datensätze (2, 3) mit unterschiedlichem Bilddateninhalt durch Bildverarbeitung bestimmbar sind, wobei die Auswertungsvorrichtung (9) Folgendes umfasst:
- eine erste Schnittstelle (10) zum Empfangen des Bilddatensatzes (1),
- eine Bildverarbeitungseinheit (11) zum Bestimmen von mindestens zwei verarbeiteten Datensätzen (2, 3), die einen anderen Bilddateninhalt als der Bilddatensatz (1) aufweisen,
- eine Auswertungseinheit (12) zum Bestimmen von quantitativen Auswertungsergebnisdaten (6), die mindestens ein dynamisches und/oder statisches Merkmal der abgebildeten Region beschreiben, durch Anwenden eines Auswertungsalgorithmus (7),
- eine zweite Schnittstelle (16) zum Bereitstellen der Auswertungsergebnisdaten (6),
wobei die Auswertungseinheit (12) umfasst:
- eine erste Untereinheit (13) zum Anwenden eines ersten Unteralgorithmus des Auswertungsalgorithmus (7) auf einen ersten der verarbeiteten Datensätze (2, 3), um ein erstes Zwischenergebnis (4) zu bestimmen, das mit dem Bilddateninhalt des ersten verarbeiteten Datensatzes (2) in Beziehung steht,
- eine zweite Untereinheit (14) zum Anwenden eines zweiten Unteralgorithmus des Auswertungsalgorithmus (7) auf einen zweiten der verarbeiteten Datensätze (2, 3), um ein zweites Zwischenergebnis (5) zu bestimmen, das mit dem Bilddateninhalt des zweiten verarbeiteten Datensatzes (4) in Beziehung steht,
- eine dritte Untereinheit (15) zum Bestimmen der quantitativen Auswertungsergebnisdaten (6) durch einen dritten Unteralgorithmus des Auswertungsalgorithmus (7), wobei der dritte Unteralgorithmus sowohl das erste als auch das zweite Zwischenergebnis (4, 5) als Eingangsdaten verwendet,
wobei der Bilddatensatz (1) ein Angiographiedatensatz ist, und die Vorrichtung **dadurch gekennzeichnet ist, dass** mindestens einer der verarbeiteten Datensätze (2, 3) ein virtuelles Nicht-Iod-Bild ist, und dass in dem Nicht-Iod-Bild eine Identifikation und Quantifizierung von Calcium durchgeführt wird, um die Identifikation und Quantifizierung von Calcium als zweites Zwischenergebnis zu ergeben.

13. Bildgebungsvorrichtung (18) mit einer Steuervorrichtung (25), die eine Auswertungsvorrichtung (9) nach Anspruch 12 umfasst.

14. Computerprogramm, das bei Ausführung des Computerprogramms auf einer Auswertungsvorrichtung (9) die Schritte eines Verfahrens nach einem der Ansprüche 1 bis 11 ausführt.

15. Elektronisch lesbares Speichermedium, auf dem ein Computerprogramm nach Anspruch 14 gespeichert ist.

## Revendications

1. Procédé mis en œuvre par ordinateur d'évaluation d'un ensemble (1) de données d'image de tomodensitométrie assisté par ordinateur à plusieurs énergies d'une région imagée, dans lequel, à partir de l'ensemble (1) de données d'image, on peut déterminer, par traitement d'image, différents ensembles (2, 3) de données traités ayant un contenu de données d'image différent et on détermine une donnée (6) de résultat d'évaluation quantitative décrivant au moins une caractéristique dynamique et/ou statique de la région imagée en appliquant un algorithme (7) d'évaluation, le procédé comprenant :
- déterminer au moins deux ensembles (2, 3) de données traités ayant un contenu de données d'image différent à partir de l'ensemble (1) de données d'image,
- appliquer un premier sous-algorithme de l'algorithme (7) d'évaluation à un premier des ensembles (2, 3) de données traités pour déterminer un premier résultat (4) intermédiaire se rapportant au contenu de données d'image du premier ensemble (2) de données traité,
- appliquer un deuxième sous-algorithme de l'algorithme (7) d'évaluation à un deuxième des ensembles (2, 3) de données traités pour déterminer un deuxième résultat (5) intermédiaire se rapportant au contenu de données d'image du deuxième ensemble (3) de données traité,
- déterminer la donnée (6) de résultat d'évaluation quantitative par un troisième sous-algorithme de l'algorithme (7) d'évaluation, dans lequel le troisième sous-algorithme utilise à la fois le premier et le deuxième résultats (4, 5) intermédiaires comme donnée d'entrée,
dans lequel l'ensemble (1) de données d'image est un ensemble de données d'angiographie et le procédé est **caractérisé en ce que** au moins l'un des ensembles (2, 3) de données traité est une image virtuelle non iodée et
dans l'image non iodée, une identification et une quantification du calcium est effectuée pour fournir l'identification et la quantification du calcium comme deuxième résultat intermédiaire.

2. Procédé suivant la revendication 1,
dans lequel au moins l'un des ensembles (2, 3) de données traité est déterminé sur la base d'une décomposition de matière et/ou sous la forme d'une image mono-énergétique.

3. Procédé suivant la revendication 2, dans lequel l'ensemble (1) de données d'image est acquis en utilisant une tomodensitométrie assistée par ordinateur à plusieurs énergies sur la base d'une source et/ou sur la base d'un détecteur, en particulier en utilisant un détecteur (24) de rayons X de comptage.

4. Procédé suivant la revendication 1 ou 2, dans lequel l'ensemble (1) de données d'image est un ensemble de données d'angiographie et au moins l'un des ensembles (2, 3) de données traité et choisi dans le groupe comprenant une image virtuelle sans contraste, une image de concentration de l'iode, une image fonctionnelle, en particulier de perfusion, une image mono-énergétique et une image virtuelle du non calcium.

5. Procédé suivant l'une des revendications précédentes, dans lequel
le premier résultat (4) intermédiaire décrit un résultat de segmentation concernant de multiples caractéristiques segmentées, dans lequel le troisième sous-algorithme assigne une donnée du deuxième résultat (5) intermédiaire à des caractéristiques segmentées pour fournir des résultats d'évaluation quantitatifs spécifiques à la caractéristique segmentée.

6. Procédé suivant la revendication 5, dans lequel les caractéristiques segmentées sont des caractéristiques anatomiques et comprennent des vaisseaux ou des segments de vaisseau d'un arbre de vaisseau et/ou des organes ou des segments d'organe.

7. Procédé suivant l'une des revendications précédentes, dans lequel
le premier résultat (4) intermédiaire comprend un arbre de vaisseau segmenté, dans lequel le troisième sous-algorithme effectue au moins une simulation d'écoulement de fluide dans l'arbre de vaisseau segmenté pour déterminer au moins un paramètre d'écoulement de fluide comme résultat d'évaluation, dans lequel la simulation est paramétrée au moins en partie en utilisant le deuxième résultat (5) intermédiaire.

8. Procédé suivant la revendication 7, dans lequel l'arbre de vaisseau segmenté est un arbre de vaisseau sanguin, le fluide est du sang et le au moins un paramètre d'écoulement du fluide comprend une réserve d'écoulement fractionnelle.

9. Procédé suivant l'une des revendications précédentes, dans lequel
au moins une partie du premier et au moins une partie du deuxième résultats (4, 5) intermédiaires sont utilisées comme donnée quantitative d'entrée à au moins une estimation de valeur de maladie du troisième sous-algorithme.

10. Procédé suivant l'une des revendications précédentes, dans lequel
le troisième sous-algorithme détermine en outre au moins une image (8) de sortie en deux dimensions visualisant la donnée (6) de résultat d'évaluation.

11. Procédé suivant la revendication 10, dans lequel on choisit sur la base du premier résultat (4) intermédiaire l'orientation et/ou le point de vue et/ou la partie de la région imagée représentée de la au moins une image (8) de sortie sur la base du deuxième ensemble (3) de données traité et/ou du deuxième résultat (5) intermédiaire.

12. Dispositif (9) d'évaluation pour évaluer un ensemble (1) de données d'image de tomodensitométrie assisté par ordinateur à plusieurs énergies d'une région imagée, dans lequel, à partir de l'ensemble (1) de données d'image, différents ensembles (2, 3) de données traités ayant un contenu de données d'image différent, sont déterminés par un traitement d'image, le dispositif (9) d'évaluation comprenant :
- une première interface (10) pour recevoir l'ensemble (1) de données d'image,
- une unité (11) de traitement d'image pour déterminer au moins les ensembles (2, 3) de données traités ayant un contenu de données d'image différent à partir du premier ensemble (1) de données d'image,
- une unité (12) d'évaluation pour déterminer une donnée (6) de résultat d'évaluation quantitative décrivant au moins une caractéristique dynamique et/ou statique de la région imagée en appliquant un algorithme (7) d'évaluation,
- une deuxième interface (16) pour fournir la donnée (6) de résultat d'évaluation,
dans lequel l'unité (12) d'évaluation comprend :
- une première sous-unité (13) pour appliquer un premier sous-algorithme de l'algorithme (7) d'évaluation à un premier des ensembles (2, 3) de données traités, afin de déterminer un premier résultat (4) intermédiaire se rapportant au contenu de données d'image du premier ensemble (2) de données traité,
- une deuxième sous-unité (14) pour appliquer un deuxième sous-algorithme de l'algorithme (7) d'évaluation à un deuxième des ensembles (2, 3) de données traité afin de déterminer un deuxième résultat (5) intermédiaire se rapportant au contenu de données d'image du deuxième ensemble (4) de données traité,
- une troisième sous-unité (15) pour déterminer la donnée (6) quantitative de résultat d'évaluation par un troisième sous-algorithme de l'algorithme (7) d'évaluation, dans lequel le troisième sous-algorithme utilise à la fois le premier et le deuxième résultats (4, 5) intermédiaires comme donnée d'entrée, dans lequel l'ensemble (1) de données d'image est un ensemble de données d'angiographie et le dispositif est **caractérisé en ce que** au moins l'un des ensembles (2, 3) de données traité est une image virtuelle non iodée et
dans l'image non iodée, une identification et une quantification du calcium est effectuée pour fournir l'identification et la quantification du calcium comme deuxième résultat intermédiaire.

13. Dispositif (18) d'imagerie ayant un dispositif (25) de commande comprenant un dispositif (9) d'évaluation suivant la revendication 12.

14. Programme d'ordinateur, qui effectue les stades d'un procédé suivant l'une quelconque des revendications 1 à 11, lorsque le programme d'ordinateur est exécuté sur un dispositif (9) d'évaluation.

15. Support de mémoire exploitable électroniquement, sur lequel un programme d'ordinateur suivant la revendication 14 est mis en mémoire.
